Europäisches Patentamt

European Patent Office

Office européen des brevets

(19)

(11) **EP 0 711 570 A1**

(12) **EUROPEAN PATENT APPLICATION**

(43) Date of publication:
15.05.1996 Bulletin 1996/20

(51) Int. Cl.$^6$: **A61M 5/142**

(21) Application number: 94117947.5

(22) Date of filing: 14.11.1994

(84) Designated Contracting States:
DE FR GB IT NL

(71) Applicant: **Siemens Elema AB**
S-171 95 Solna 1 (SE)

(72) Inventor: **Slettenmark, Bruno**
S-175 60 Järfälla (SE)

(54) **Method and device for internal cleaning of an implanted infusion system**

(57)    In a method for internal cleaning of an implanted infusion system, comprising an infusion pump connected on its outlet side to a catheter (12) via a nonreturn valve (14), a flushing port (2) also being provided downstream the nonreturn valve and upstream the catheter, a first flow (Q1) of cleaning fluid is passed through the infusion pump and the nonreturn valve. A second flow (Q2) of secondary fluid is supplied through the flushing port, and a third fluid flow (Q3) is discharged through the flushing port. A device for such a cleaning comprises pumping means connected to the flushing port for supplying the second flow of secondary fluid, intended for diluting or otherwise modifying the cleaning fluid passing through the infusion pump and the nonreturn check valve, and for discharging the third flow.

FIG 1A

FIG 1B

EP 0 711 570 A1

Printed by Rank Xerox (UK) Business Services
2.11.6/3.4

## Description

The present invention relates to a method and a device for internal cleaning of an implanted infusion system, comprising an infusion pump connected on its outlet side to a catheter via a nonreturn valve, a flushing port also being provided downstream the nonreturn valve and upstream the catheter, a first flow of cleaning fluid being passed through the infusion pump and the nonreturn valve.

Implantable infusion systems or infusers deliver medication or drugs, usually in liquid form, such as an insulin solution, to the patient. For example, such an infuser can consist of a.o. a drug reservoir, a micropump with a nonreturn valve and a catheter. One problem here is that medication can become deposited on the interior surfaces of a.o. the pump and the nonreturn valve. A reduced volume delivered per pump stroke, valve leakage etc. are some of the disruptions which then can occur. If such a disruption occurs, the deposits in the system can be dissolved and the pump performances restored. For example, the reservoir can be filled with some cleaning or washing liquid which dissolves the deposits in question and allow the liquid to pass through the pump by activating the pump funktion. Since the washing liquid can be aggressive, toxic or unapproved as a drug etc., it is important to prevent the liquid from being pumped out of the catheter and coming into contact with body tissues. Allowing dissolved drug deposits to enter the body is further inappropriate, since this can cause immune reactions. For example, denatured insulin in the aodominal cavity can give rise to the formation of antibodies, and the patient can ultimately develop resistance to insulin. Therefore no flow of washing liquid out of the catheter is desirable during the washing operation. Nor is an influx of body fluid into the catheter desirable. Body fluids contain a.o. proteins which can contribute to catheter blockage and contamination of the so-called flushing port preceding the catheter. The presence of body fluid in this part of the infuser can have an adverse effect on the drug and on the life of the infuser.

As regards the insulin pumps, the problem has hitherto been ignored and the patient has been exposed to unacceptable risks by pumping corrosive fluids out into the aodominal cavity of the patient or the following procedure has been used:

A drain, consisting of a liquid-filled flexible tube, is connected via a cannula to the flushing port with its other end positioned on a level lower than the tip of the catheter. As a result of the siphon effect, any free fluid in the abdominal cavity then flows back through the catheter out into the drain. Pumped washing liquid, often pumped with intermittent pump strokes, will then be distributed between the catheter and the drain in a ratio determined by the transient flow resistances. In favorable conditions, the quantity of washing liquid exiting through the catheter will be returned to the drain because of the retrograde siphon flow of body fluid in the catheter. However, the method has several major weaknesses.

Usually there is very little free liquid in the abdominal cavity, and any liquid there is highly viscous. Since the interior lumen of the catheter is very small, typically 0.3 mm in diameter, an appreciable retrograde flow is uncertain. Moreover, there is a risk that the tip of the catheter presses against body tissue in the abdominal cavity, thereby preventing any retrograde flow. However, it will allow forward flow because an increase in the catheter pressure momentarily lifts the catheter tip. So under certain circumstances, a valve function can develop in the catheter orifice, and there could be an outflow of hazardous substance into the abdominal cavity, despite the drainage provided.

Thus, the prior art methods provide neither desired control of the procedure nor patient safety.

The object of the present invention is to make washing of the interior of infusion systems with aggressive fluids possible without risk to the patient or to the infusion system, in already implanted infusion systems and in future systems equipped with a flushing port.

This object is achieved with a method and a device of the kind defined in the introductory part with the characterizing features set forth in claims 1 and 9 respectively.

Thus, the invention is applicable to infusion systems with a so-called flushing port downstream the pump valve but upstream from the catheter. A check can be performed with a cannula and syringe through this flushing port to ensure that the catheter is open an if this is not the case, an applied pressure/flow will often re-open the catheter and restore the function.

The invention will now be described in greater detail, with reference to accompanying drawings on which

FIG. 1A illustrates the principles underlying the invention;
FIG. 1B shows a double-lumen cannula which can be used with the invention;
FIGS. 2A, 2B and 3 show different embodiments of pumping means in the device according to the invention;
FIG. 4 illustrates the use of the double-lumen cannula in FIG. 1B and the pumping means in FIGS. 2B; and
FIGS. 5A and 5B show two alternative versions of the pumping means in the device according to the invention.

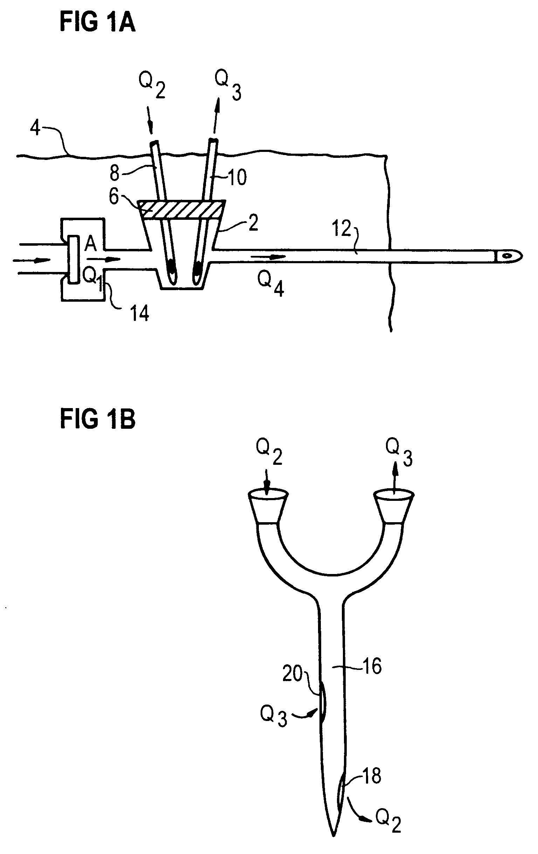

The cleaning or washing liquid which passes the pump and the valve 14 at the flow of Q1 and passes into the flushing port 2, see FIG. 1A, is mixed in the flushing port 2 with another secondary liquid with a flow of Q2 >> Q1 which is continuously injected with a cannula 8 inserted through the skin 4 and the flushing septum 6. The liquid mixture is extracted with a flow Q3, through another cannula 10, through the flushing septum 6, the flow Q3 being in a very exact ratio to Q1 and Q2. In this way, the desired magnitude of the resultant outflow Q4 of the liquid mixture through the catheter 12 can be controlled very exactly.

Generally, the following applies:

$Q_1 + Q_2 = Q_3 + Q_4$ and
$Q_2, Q_3 \gg Q_1$

Usually, $Q_4 = 0$ is desired, whereby $Q_3 = Q_2 + Q_1$, but in certain instances either a very small but positive outflow ($Q_4 > 0$) is desirable, whereby $Q_1 + Q_2 > Q_3$, or a very small inflow ($Q_4 < 0$), whereby $Q_3 > Q_2 + Q_1$. Thus, no liquid will enter the abdominal cavity ($Q_4 = 0$, or $Q_4 < 0$) or a very small, well-defined quantity of the strong washing liquid, which is diluted (typically 100-fold) to a harmless concentration or a chemically neutralized washing liquid ($Q_4 > 0$) enters the abdominal cavity.

The secondary liquid or fluid can be a) neutral, e.g. distilled water, just to physically dilute the washing liquid or fluid to a harmless concentration, or b) chemically active to neutralize the washing liquid or fluid for the purpose of making it harmless to body tissue. For example, the washing liquid or fluid can be a NaOH solution, and the secondary liquid or fluid can in case a) be sterile water and in case b) a buffer solution which effectively reduces the pH value of the mixture, an acid solution etc.

The two cannulas 8, 10 for secondary and transport fluids can advantageously be replaced with a double-lumen cannula 16 (FIG. 1B) to facilitate the whole procedure and reduce the trauma to both the patient and the flushing septum (a small area is unable to withstand many punctures, is difficult to locate in a stout patient etc.). Here, a relatively large distance between the orifices 18, 20 for the flows $Q_2$ and $Q_3$ respectively is desired to avoid any flow occurring straight from the orifice 18 to the orifice 20.

The device according to the invention makes it possible to retain, with great accuracy, a given ratio between the flows $Q_1$, $Q_2$ and $Q_3$ with simple equipment.

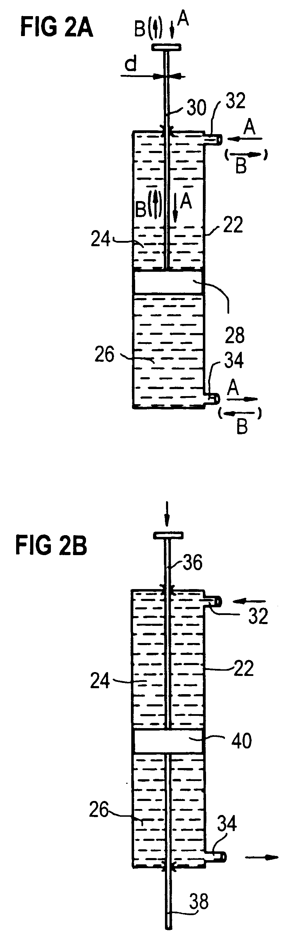

In FIG. 2A is shown a first embodiment of the pump means in the device according to the invention in the form of a syringe with a cylinder 22 in which a piston 28 is displaceable. The piston 28 can be manipulated with a piston rod 30. Each end of the cylinder 30 is provided with openings 32, 34 for the intake and pumping out of fluid as the piston 28 is moved.

When the piston 28 is moved in direction A in FIG. 2A, after the syringe has been connected to the flushing port 2, fluid is pumped out through the opening 34 at the same time as fluid is drawn in through the opening 32. Then the outflow Qout must be greater than inflow Qin into the cylinder 22 and the difference in flow magnitudes is determined by the dimension of the piston rod 30.

When the piston is moved in direction B in the FIG., there is an outflow Qout through the opening 32 and an inflow Qin through the opening 34. Here, $Q_{in} > Q_{out}$.

In FIG. 2B is shown an alternative version with a similar piston rod 36, 38 attached to either side of the piston 40. In this case, the flows $Q_{out}$, $Q_{in}$ resulting when the piston 40 is moved are equal.

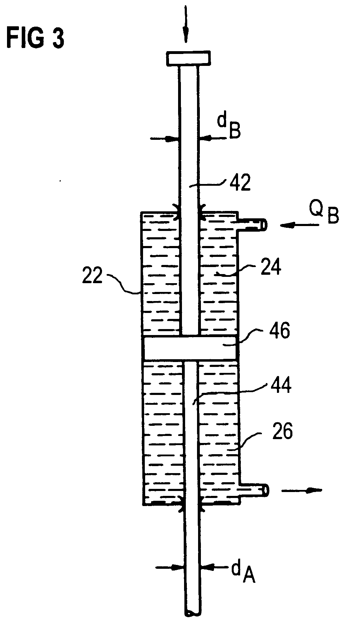

In FIG. 3 is shown another alternative version with two piston rods 42, 44, the two piston rods 42, 44 having different dimensions. If the diameter of the piston rod 42 is denoted by $d_B$ and the diameter of the piston rod 44 by $d_A$, the following conditions will apply to the flows $Q_A$ and $Q_B$ resulting when the piston 46 is moved downwards in the FIG.

If $d_A < d_B$, then $Q_A > Q_B$,
if $d_A > d_B$, then $Q_A < Q_B$ and
if $d_A = d_B$, then $Q_A = Q_B$.

Thus, desired flows can be set by the appropriate choice of pistol rod dimensions. So the condition $Q_3 = Q_2 + Q_1$, i.e. $Q_4 = 0$, can be realized through appropriate choice of $d_A$ and $d_B$, whereby $Q_B$ corresponds to $Q_3$ and $Q_A$ to $Q_2$.

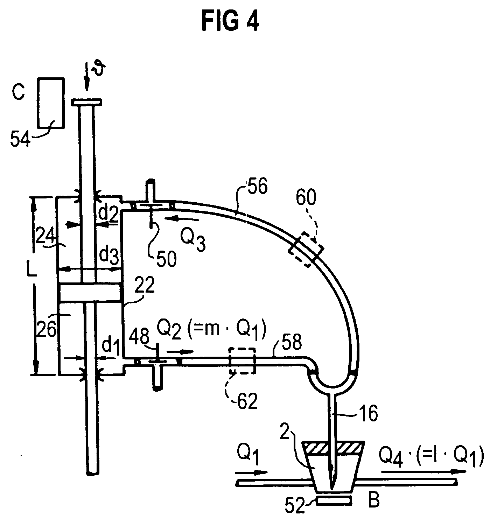

In FIG. 4 is shown the syringe in FIG. 2B connected by the flexible tubes 56, 58 and a double-lumen catheter 16 to the flushing port 2.

At a given value for the flow $Q_1$, a dilution factor m and the flow $Q_4$ (cf. FIG. 4), the parameters $d_1, d_2, d_3$ and the feed velocity v can be selected and calculated respectively. One dimensioning example is as follows:

Dimensioning example:

Assume that $Q_1 = 10^{-10}$ m³/s (corresponding to the maximum flow of the pump of 1 µl/10 s, so-called bolus dose. There is one pump stroke per 10s, and each stroke gives 1 µl).
Duration = 4,000 s, resulting
in a total quantity of 0.4 ml.

Further assume that the dilution factor m = 100, i.e. $Q_2 = m \cdot Q_1$.
Also assume that $Q_4 = l \cdot Q_1$, where l is an optional factor, positive, negative or = 0.

Calculations give the following relationship:

$$d_1 = \left[ \frac{\frac{1-l}{m} \cdot d_3{}^2 + d_2{}^2}{1 + \frac{1-l}{m}} \right]^{\frac{1}{2}}$$

$$v = \frac{4Q_1}{\pi} \cdot \left[ \frac{1 + m - l}{d_3{}^2 - d_2{}^2} \right]$$

If we now select
l = 0 (i.e. $Q_4 = 0$)
$d_3 = 3 \cdot 10^{-2}$ m
$d_2 = 6 \cdot 10^{-3}$ m
we get
$d_1 = 6.68 \cdot 10^{-3}$ m
$v = 14.9 \cdot 10^{-6}$ m/s

The length of the syringe is $L \geq 7 \cdot 10^{-2}$ m (stroke length $\geq 6 \cdot 10^{-2}$ m).

To attain the intended accuracy, it is essential for both chambers of the syringe 24, 26 to be filled, free from air, at the start of the procedure. Otherwise, the fluid medium will not be incompressible and the $Q_2/Q_3$ ratio will not remain reliably constant. In order to facilitate air-free filling, the syringe can be equipped with e.g. a three-way cock 48, 50 according to FIG.4 or some similar means through which the system can be de-aired.

In the following two examples are given of how to effectively clean a catheter.

The infusion pump is delivering the flow $Q_1$ which is comprised of first 0,4 ml buffer solution followed by 0,4 ml of 50 mM NaOH and finally 0,6 ml buffer solution, that is a total volume of liquid of 1,4 ml. This liquid is continuously mixed with the secondary fluid supplied into the flushing port by a syringe and sucked out again such that the flow out through the catheter is zero or approximately zero, that is $Q_2 \approx Q_3$ or $Q_A \approx Q_B$ in figures 2 - 4. The secondary fluid can have a purely diluting effect, but is preferably buffering, which gives a better result. For several reasons it is desirable to rinse with the secondary fluid during the whole procedure and not only in the phase during which NaOH is supplied. With a dilution factor m = 100 the volume of the syringe will be 140 ml which is an impractically large syringe. In practice it has been found however, that a dilution factor m = 28 which corresponds to a syringe volume of 39 ml, gives excellent results in a device with $d_A = d_B$.

The flow rate through the infusion pump is, however, extremely low, about 0,1 $\mu$l/s, and the flow of secondary fluid is rather low too for a dilution factor m = 28 or even m = 100. Thus, there will be a very small flow in the flushing port and the agitating effect will be moderate. If it comes to the worse through unfortunate positioning and/or design of the double lumen cannula used for the flushing, in the case with a small fluid volume flowing out through the catheter, that is in the case $d_A = d_B$, it could theoretically be no mixing or the mixing could be inadequate, such that the flows in the flushing port would be essentially laminar and a too high concentration of NaOH could be discharged through the catheter.

To avoid this risk and enhance the mixing effect in the flushing port, with the use of a syringe of reasonable size and in the case with $d_A = d_B$ the following procedure can be used.

The piston of the secondary fluid syringe is moved with an essentially constant velocity $v_1$, first in forward direction with this velocity for a time $t_1$, whereupon the piston is moved with the same velocity in the opposite direction for a time $t_2$ which is shorter than the time $t_1$. Thereafter the piston is moved in the forward direction for another period $t_1$ followed by movement in the opposite direction during a time $t_2$ and so on.

The time period $t_1$ can have a length from a few seconds to several minutes and the velocity of the piston $v_1$ is relatively high to get a flow in the flushing port which is e.g. 1000 times the flow from the infusion pump, that is m = 1000, or even higher.

The flow of the pump is 0,1 $\mu$l/s as mentioned above and for a secondary flow of 100 $\mu$l/sec (m = 1000) an effective mixing will be obtained in the flushing port for the geometric dimensions in question.

The average velocity $\bar{v}$ is given by

$$\bar{v} = \frac{v_1 \cdot t_1 - v_1 \cdot t_2}{t_1 + t_2} = v_1 \cdot \frac{t_1 - t_2}{t_1 + t_2}$$

and adapted such that the piston of the syringe will reach its end position at the same time or after the termination of the primary flow $Q_1$.

Another method of operating the device shown in e.g. figure 2B is to move the piston of the secondary fluid syringe from one end postion to the other and then back to the first end position and so on. The piston is moved with the velocity which corresponds to the desired dilution, e.g. m = 1000.

Both in this mode of operation and the previously described one the concentration of primary fluid will increase during the operation, since the secondary fluid is re-used for dilution of the primary fluid for a plurality of times. Independent of the velocity of the piston, and consequently independent of the value of the parameter m, the concentration of NaOH at the end of the procedure will be $\leq 0,4$ ml 50 mM NaOH in 39 ml fluid volume in the secondary fluid syringe, that is the concentration will be

$$\frac{0,4}{39} = 0,0103$$

ml caustic solution/ml fluid.

In the case with $d_A = d_B$ a total fluid volume of 1,2 ml will pass through the catheter out into the abdominal cavity. The concentration c in this fluid will be

$$c \leq \frac{\left[ m \cdot \dfrac{0,4}{39} + 1 \right]}{m + 1}$$

ml lut/ml fluid
and for m = 1000
c = 0,0112 ml caustic solution/ml fluid
which corresponds to a dilution of one part caustic solution per 88 parts secondary fluid. This dilution fulfils by a very wide margin the requirement for a harmless pH value of the fluid reaching the abdominal cavity, provided that as secondary fluid is used e.g. the "dilution buffer" made by the company Hoechst.

Thus, with the above described examples of mode of operation safe mixing is obtained in the flushing port for every operating condition with a reasonable size of the secondary fluid syringe suitable for commercially available syringe pumps, and it is completely secured that the small volume of fluid which is passing out into the abdominal cavity is harmless.

Since the consequences could be grave if the device failed to work as intended, the system can be supplemented with those control functions which are deemed necessary to assure reliable function. Some examples are:

a. A device 52 which monitors the tip of the cannula to determine whether it is in the correct position inside the flushing port throughout the entire procedure.

B. A position sensor/speedometer 54 which checks that the piston rod is moving in the intended manner.

C. A flow sensor, especially for the return flow $Q_3$ but eventually also for the inflow $Q_2$. If there is no return flow $Q_3$ for some reason, e.g. because the cannula orifice is blocked or the like, the flow $Q_1 + Q_2$ will pass out into the catheter. This is not necessarily dangerous but is inappropriate in any event. The flow sensor 60 in FIG. 4 ensures that $Q_3$ is close to the desired value. The sensor 60 can lie in the fluid path and does not have to be sterile, since the return flow does not have to be sterile. The sensor 60 can be a commercial mass flow meter or the like. (If the flow $Q_3$ is interrupted, the pressure in the interior of the syringe will drop to the vapor pressure of the fluid at the prevailing temperature, whereupon vapor bubbles will form, and the piston movement will continue, although against a higher resistance.)

If the flow $Q_2$ stops or is delivered to the wrong place, body fluid will be drawn back through the catheter (i.e. $Q_4 = -Q_3$ ) and be intermixed and transport $Q_1$ out in the return flow. Cleaning fluid will not enter the aodominal cavity of the patient. If there is no body fluid to extract, the sensor 60 will detect a small flow and issue an alarm signal. An inflow gauge 62 may still be desired/necessary (drawing of body fluid into the catheter/flushing port is a.o. undesirable). The gauge 62 must be sterile if it is to lie in the flow of fluid. The flow $Q_2$ can be measured outside the fluid pathway, e.g. with an autocorrelation meter, an ultrasonic doppler meter, a laser doppler meter, meters based on measurement of the Hall effect (electrically conductive fluid) etc. A non-homogenous fluid, to which e.g. very small particles are added, might possibly be needed for a doppler meter in order to give a signal diffused back with sufficient strength.

The equipment can advantageously comprise sterilized disposable articles made of rubber and plastic, such as commercially available syringes. Piston operation can be accomplished with commercially available infusion equipment with appropriate performances. One major advantage with the equipment is that one and the same piston and piston rod provide both outflow and inflow. The momentary $Q_2/Q_3$ ratio is independent of fluctuations in the velocity v. In addition, a slow spatial variation in the diameter $d_3$ along the length of the syringe 22 only affects the $Q_2/Q_3$ ratio slightly or not at all.

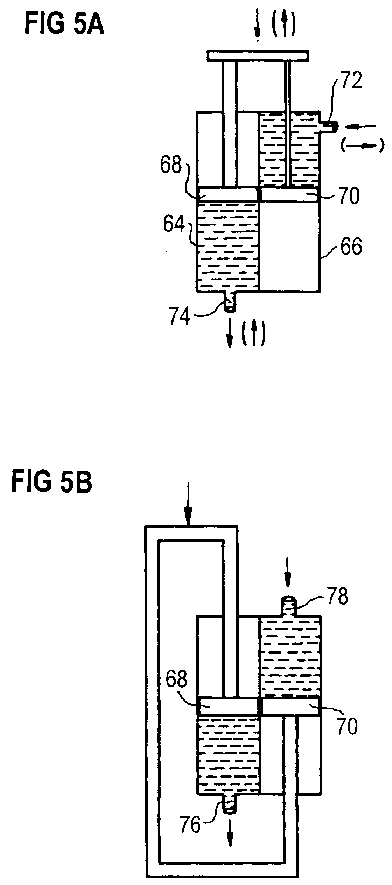

FIGS. 5A and 5B show two possible versions which, however, do not exhibit said advantages.

In FIG. 5 is thus shown one version of pumping means with two parallel cylinders 64, 66, each with a piston 68, 70, said pistons 68, 70 being interconnected for joint movement. The cylinders 64, 66 are devised with openings 72, 74 and 76, 78 respectively at opposite ends, whereby a flow of fluid is pumped out through one of the openings, and a flow of fluid is drawn in through the other opening, or vice-versa depending on the direction of movement of the pistons 68, 70.

The flow Q1 of cleaning fluid is very small (about $10^{-10}$ m³/s), whereas the flows $Q_2$ and $Q_3$ can be e.g. 100 times larger. A temporary (or constant) imbalance of e.g. 5% between the flows $Q_2$ and $Q_3$ will then result in a large, undesirable inflow/outflow $Q_4 = 5 \cdot Q_1$. Such an imbalance can occur in the devices in FIG. 5 if extraordinary measures are not taken, but not in the device in FIG. 4. Even if a relatively simple equipment is used with moderately low tolerance requirements and even if the piston is made of rubber, and is thus capable of deformation and possibly jerky movement, the piston is incompressible and does not change the $Q_2/Q_3$ ratio. The accuracy of the desired flow $Q_4$ is just as good or better than the accuracy attainable with very expensive and complex equipment which would not be able to utilize sterilized disposable articles.

The invention has been described above as applied to a system with the catheter opening into the abdominal cavity. However, the invention is obviously applicable to systems with the catheter opening into other sites in the body, e.g. into blood vessels.

The above-mentioned secondary fluid or liquid can be a diluent fluid for purely physical dilution of the cleaning fluid. However, such dilution is often inadequate in rendering the diluted fluid harmless to body tissue, and therefore the secondary fluid often consists of a buffer solution. In theory, an acid could also be used as secondary fluid.

**Claims**

1. Method for internal cleaning of an implanted infusion system, comprising an infusion pump connected on its outlet side to a catheter via a nonreturn valve, a flushing port also being provided downstream the nonreturn valve and upstream the catheter, in which method a first flow of cleaning fluid is passed through the infusion pump and the nonreturn valve, characterized in that a second flow of secondary fluid is supplied through the flushing port, and a third flow of fluid is discharged through the flushing port.

2. Method according to of claim 1, characterized in that the second and third flows through the flushing port are much larger than the first fluid flow through the infusion pump and the nonreturn valve.

3. Method according to claim 1 or 2, characterized in that the second and third flows through the flushing port are of equal magnitude.

4. Method according to claim 1 or 2, characterized in that the third flow is equal to the sum of the first flow and the second flow.

5. Method according to claim 1 or 2, characterized in that the sum of the first and second flow is larger than the third flow.

6. Method according to any of claims 1 or 2, characterized in that the sum of the first and second flow is smaller than the third flow.

7. Method according to any of claims 1 - 6, characterized in that the secondary fluid is a neutral fluid, such as sterile water, which acts by physical dilution.

8. Method according to any of claims 1 - 6, characterized in that the secondary fluid is a chemically active fluid for neutralizing or otherwise modifying the cleaning fluid.

9. Method according to any of claims 1 - 8, **characterized** in that the directions of the second and third flows are reversed at least twice during the cleaning procedure.

10. Device for internal cleaning of an implanted infusion system, comprising an infusion pump connected on its outlet side to a catheter via a nonreturn valve, a flushing port being provided downstream the nonreturn valve and upstream the catheter, characterized in that pumping means are connected to the flushing port to supply a second flow of a secondary fluid, for diluting or otherwise modifying a first flow of cleaning fluid passing through the infusion pump and the nonreturn valve, and for discharging a third flow of fluid.

11. Device according to claim 10, characterized in that the pumping means comprise a syringe in the form of a cylinder in which a piston is displaceable, said cylinder being devised with openings on either side of the piston.

12. Device according to claim 11, characterized in that the piston can be manipulated by a piston rod running from one side of the piston in the longitudinal direction of the cylinder and out through one end wall of the cylinder.

13. Device according to claim 11, characterized in that a piston rod runs from either side of the piston out through the end walls of the cylinder.

14. Device according to claim 10, characterized in that the pumping means comprise two cylinders, each having a piston, which pistons are interconnected for joint movement, and in that the space on one side of the piston of the first cylinder is devised with an opening for discharging and drawing in fluid in this space by displacing the piston of the cylinder, and the space on the other side of the piston of the second cylinder is devised with an opening for drawing fluid into this space by displacing the piston of the cylinder movements during the discharge phase of the first cylinder and for discharging fluid during the drawing in phase of the first cylinder.

15. Device according to claims 13 or 14, characterized in that the piston rods have the same thickness.

16. Device according to claim 13 or 14, characterized in that the piston rods have different thicknesses.

17. Device according to any of claims 10 - 16, characterized in that a double-lumen cannula is introducible into the flushing port to respectively supply and remove the second and third flows.

18. Device according to any of claims 10 - 17, characterized in that a sensor is arranged between the pumping means and the flushing port in order to measure the third flow.

19. Device according to any of claims 10 - 18, characterized in that a sensor is arranged between the pumping means and the flushing port in order to measure the second flow.

20. Device according to any of claims 11 - 19, characterized in that the means are provided to sense when the tip of the syringe or cannula is in the correct position in the flushing port during the cleaning procedure.

21. Device according to any of claims 12 - 20, characterized in that position and/or velocity gauges are arranged to check that the piston is moving in the intended manner.

**FIG 1A**

$Q_2$  $Q_3$

4

8  10

6

A  2  12

$Q_1$

14  $Q_4$

**FIG 1B**

$Q_2$  $Q_3$

20

$Q_3$  16

18

$Q_2$

**FIG 2A**

**FIG 2B**

FIG 3

## FIG 4

**FIG 5A**

**FIG 5B**

European Patent
Office

**EUROPEAN SEARCH REPORT**

Application Number

EP 94 11 7947.5

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.6) |
|---|---|---|---|
| A | US, A, 4573994 (ROBERT E. FISCHELL ET AL), 4 March 1986 (04.03.86) * column 3, line 37 - line 42 * | 1-21 | A61M 5/142 |
| | ----- | | |
| | | | **TECHNICAL FIELDS SEARCHED (Int. Cl.6)** |
| | | | A61M |

The present search report has been drawn up for all claims

| Place of search | Date of completion of the search | Examiner |
|---|---|---|
| STOCKHOLM | 13 February 1995 | MAY HALLNE |

CATEGORY OF CITED DOCUMENTS

X : particularly relevant if taken alone
Y : particularly relevant if combined with another document of the same category
A : technological background
O : non-written disclosure
P : intermidiate document

T : theory or principle underlying the invention
E : earlier patent document, but published on, or after the filing date
D : document cited in the application
L : document cited for other reasons
............................................
& : member of the same patent family, corresponding document

EPO FORM 1503 03.82 (P0401)